# EUROPEAN PATENT APPLICATION

(11) **EP 4 671 231 A1**
(43) Date of publication of application: **31.12.2025**
(21) Application number: 24760268.3
(22) Date of filing: 16.02.2024
(51) Int. Cl.: C07C 68/06, C07B 61/00, C07C 68/04, C07C 69/96

(54) **METHOD FOR PRODUCING CARBONIC ACID DIESTER**

(30) Priority: 22.02.2023 JP 2023025910
(71) Applicant: National Institute of Advanced Industrial Science and Technology, Chiyoda-ku Tokyo 100-8921 (JP); Tosoh Corporation, Yamaguchi 746-8501 (JP)
(72) Inventor: TAKEUCHI, Katsuhiko, Tsukuba-shi, Ibaraki 305-8565 (JP); CHOI, Jun-Chul, Tsukuba-shi, Ibaraki 305-8565 (JP); FUKAYA, Norihisa, Tsukuba-shi, Ibaraki 305-8565 (JP); MATSUMOTO, Kazuhiro, Tsukuba-shi, Ibaraki 305-8565 (JP); KOIZUMI, Hiroki, Tsukuba-shi, Ibaraki 305-8565 (JP); YAMAMOTO, Toshihide, Yokkaichi-shi, Mie 510-8540 (JP); INOUE, Yoshiaki, Yokkaichi-shi, Mie 510-8540 (JP); YOSHIDA, Takumu, Yokkaichi-shi, Mie 510-8540 (JP); INUI, Akira, Yokkaichi-shi, Mie 510-8540 (JP); NAKASHIGE, Makoto, Shunan-shi, Yamaguchi 746-8501 (JP); HIROTA, Junya, Shunan-shi, Yamaguchi 746-8501 (JP); HASHIZUME, Yusuke, Shunan-shi, Yamaguchi 746-8501 (JP); YAMASAKI, Shun, Shunan-shi, Yamaguchi 746-8501 (JP); HAMURA, Satoshi, Tokyo 104-8467 (JP); MASUDA, Takahiro, Shunan-shi, Yamaguchi 746-8501 (JP)
(74) Representative: Novagraaf International SA
(86) International application number: PCT/JP2024/005525
(87) International publication number: WO 2024/176969

(57) **Abstract**

A method for producing a carbonic acid diester, including a reaction step of reacting a carbonic acid monoester salt with an alkoxysilane in the presence of one or more catalysts selected from the group consisting of a cerium catalyst and a tin catalyst.

## Description

### TECHNICAL FIELD

The present disclosure relates to a method for producing a carbonic acid diester.

### BACKGROUND ART

Carbonic acid diesters are widely used as solvents such as electrolytes, alkylating agents, carbonylating agents, gasoline additives, diesel fuel additives, and raw materials for polymers such as polycarbonates.

As a method for producing carbonic acid diesters, a method of reacting phosgene with an alcohol is widely known and has also been industrialized. However, this method has problems such as corrosion of equipment due to by-produced hydrochloric acid, and the necessity of using highly toxic and corrosive phosgene. Therefore, the development of technologies for producing carbonic acid diesters without using phosgene has been promoted.

Patent Document 1 discloses a method for producing an organic carbonate by reacting an alcohol, carbon dioxide, and an alkyl halide in an ionic liquid.

Non-Patent Document 1 discloses a method for producing dimethyl carbonate by dissolving an ionic liquid saturated with carbon dioxide, then adding methyl iodide, and performing electrochemical reduction using an NPC-Ag electrode.

### PRIOR ART DOCUMENTS

### PATENT DOCUMENTS

Patent Document 1: CN 111362800 A

### NON-PATENT DOCUMENTS

Non-patent Document 1: Chinese Chemical Letters, 2010, 987-990

### SUMMARY OF INVENTION

### TECHNICAL PROBLEM

Although the methods described in Patent Document 1 and Non-Patent Document 1 do not require the use of phosgene, they respectively require the use of methyl iodide and an alkyl halide as sacrificial reagents. Since these sacrificial reagents have a large environmental burden, reducing their usage has become a social issue in recent years, as efforts toward environmental problems are increasingly emphasized.

An object of the present disclosure is to provide a novel method for producing a carbonic acid diester without using sacrificial reagents such as alkyl halides and without phosgene.

### SOLUTION TO PROBLEM

As a result of diligent studies by the present inventors to solve the above problems, it was found that a carbonic acid diester can be produced by reacting a carbonic acid monoester salt with an alkoxysilane in the presence of a catalyst, without using sacrificial reagents such as alkyl halides and without phosgene. That is, the present disclosure includes the followings.
[1] A method for producing a carbonic acid diester, comprising a reaction step of reacting a carbonic acid monoester salt with an alkoxysilane in the presence of one or more catalysts selected from the group consisting of a cerium catalyst and a tin catalyst.
[2] The method for producing the carbonic acid diester according to [1], wherein the carbonic acid monoester salt is a compound represented by general formula (1), the alkoxysilane is a compound represented by general formula (2), and the carbonic acid diester is a compound represented by general formula (3): 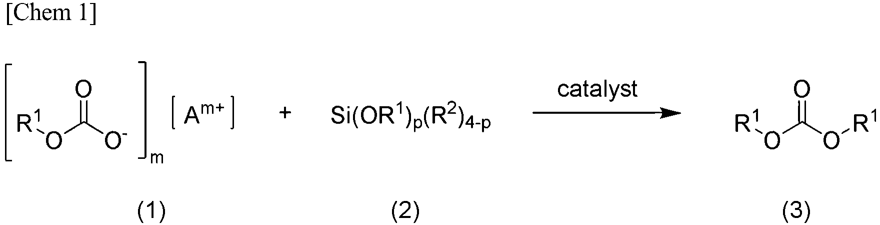 wherein in the general formulae, R¹ each independently is a substituted or unsubstituted hydrocarbon group; R² each independently is a substituted or unsubstituted hydrocarbon group; A^{m+} is an m-valent cation derived from a base represented by A; m is 1 or 2; p is an integer of 1 to 4; and when p is 1 or 2, the plurality of R² may be linked to each other to form a ring.
[3] The method for producing the carbonic acid diester according to [2], wherein R¹ is a substituted or unsubstituted aliphatic hydrocarbon group.
[4] The method for producing the carbonic acid diester according to [2] or [3], wherein p is 2 to 4.
[5] The method for producing the carbonic acid diester according to any one of [1] to [4], wherein the reaction step is performed under a temperature condition of 80°C or more and 250°C or less.
[6] The method for producing the carbonic acid diester according to any one of [1] to [5], wherein the cerium catalyst is cerium (IV) oxide.
[7] The method for producing the carbonic acid diester according to any one of [1] to [6], wherein the tin catalyst is one or more selected from the group consisting of a dialkyltin bis(aliphatic monocarbonate), and a dialkyltin oxide.
[8] The method for producing the carbonic acid diester according to any one of [1] to [7], comprising, prior to the reaction step, a carbonic acid monoester salt production step of contacting an alcohol, a base, and a carbon dioxide-containing gas,
   wherein a ratio of partial pressure P_{CO2} of carbon dioxide gas in the carbon dioxide-containing gas to total pressure Pₜ of the carbon dioxide-containing gas is 0.0001 or more and 1.00 or less.
[9] The method for producing the carbonic acid diester according to [8], wherein the partial pressure P_{CO2} of carbon dioxide gas in the carbon dioxide-containing gas is less than 0.100 MPa.

### ADVANTAGEOUS EFFECTS OF INVENTION

According to the present disclosure, a novel method for producing a carbonic acid diester can be provided without using sacrificial reagents such as alkyl halides and without phosgene.

### DESCRIPTION OF EMBODIMENTS

The present disclosure will be described in detail below. However, the description of the constituent requirements described below is an example (typical example) of embodiments of the present disclosure, and the present disclosure is not limited to these contents but can be implemented with various modifications within the scope of its gist.

In the present disclosure, a description such as "one or more selected from the group consisting of X, Y, and Z" means any one of X, Y, Z, a combination of X and Y, a combination of X and Z, a combination of Y and Z, or a combination of X, Y, and Z.

In the present disclosure, unless otherwise specified, descriptions of numerical ranges such as "X or more and Y or less" and "X to Y" mean numerical ranges including the lower limit and upper limit, which are endpoints.

In the present disclosure, a description such as "X such as x1, x2, and x3" refers to x1, x2, and x3 as examples of X, and does not mean that X is limited to x1, x2, and x3.

A method for producing a carbonic acid diester according to one embodiment of the present disclosure includes a reaction step of reacting a carbonic acid monoester salt with an alkoxysilane in the presence of one or more catalysts selected from the group consisting of a cerium catalyst and a tin catalyst. The production method according to the present embodiment is a method for producing a carbonic acid diester using a carbonic acid monoester salt as a raw material, and does not require the use of phosgene as a raw material. Furthermore, in the production method according to the present embodiment, the reaction proceeds under relatively low temperature conditions, and a carbonic acid diester is produced, even without using a sacrificial reagent. That is, the method for producing a carbonic acid diester according to the present embodiment has high industrial utility value because it is an environmentally friendly production method that can reduce production costs and has excellent energy efficiency.

According to the production method of the present embodiment, a carbonic acid diester can be produced without using sacrificial reagents and phosgene. However, in the present disclosure, "without using sacrificial reagents and phosgene" means that the use of sacrificial reagents and phosgene is not required, and although it is preferable not to use sacrificial reagents and phosgene, it is not intended to exclude all embodiments in which either one or both of sacrificial reagents and phosgene are used. For example, if the production method according to the present embodiment includes a step of producing a carbonic acid monoester salt, such a step may be a step of producing a carbonic acid monoester salt using phosgene as a raw material.

### 1. Reaction Step

In the reaction step, a carbonic acid monoester salt reacts with an alkoxysilane in the presence of one or more catalysts selected from the group consisting of a cerium catalyst and a tin catalyst, to produce a carbonic acid diester.

### 1-1. Carbonic Acid Monoester Salt

The carbonic acid monoester salt is not particularly limited and can be appropriately selected according to the target carbonic acid diester. The carbonic acid monoester salt may be used alone, or two or more types may be used in combination in any combination and ratio.

In the present embodiment, the carbonic acid monoester salt is preferably a compound represented by general formula (1).

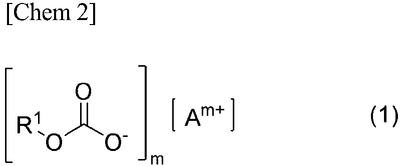

### (R¹)

R¹ each independently is a substituted or unsubstituted hydrocarbon group.

In the present disclosure, hydrocarbon groups include aliphatic hydrocarbon groups and aromatic hydrocarbon groups. Aliphatic hydrocarbon groups are not limited to linear hydrocarbon groups, and may have a branched structure, may have carbon-carbon unsaturated bonds, or may have a cyclic structure. Further, aromatic hydrocarbon groups may be monocyclic, polycyclic, or fused cyclic, and may be heterocyclic aromatic hydrocarbon groups.

The number of carbon atoms in the hydrocarbon group represented by R¹ is not particularly limited. When the hydrocarbon group is an aliphatic hydrocarbon group, the number of carbon atoms is usually 1 or more, preferably 2 or more, and usually 30 or less, preferably 24 or less, more preferably 12 or less, and still more preferably 8 or less. That is, preferred ranges for the number of carbon atoms in the aliphatic hydrocarbon group include 1 to 30, 1 to 24, 2 to 12, and 2 to 8.

When the hydrocarbon group is an aromatic hydrocarbon group, the number of carbon atoms is usually 3 or more, preferably 6 or more, and usually 30 or less, preferably 24 or less, and more preferably 20 or less. That is, preferred ranges for the number of carbon atoms in the aromatic hydrocarbon group include 3 to 30, 6 to 24, and 6 to 20.

In the present disclosure, when a hydrocarbon group has a substituent, the number of carbon atoms indicated for the hydrocarbon group is deemed to include the number of carbon atoms in the substituent.

Examples of the unsubstituted aliphatic hydrocarbon group include: an alkyl group such as methyl group, ethyl group, n-propyl group, isopropyl group, n-butyl group, sec-butyl group, tert-butyl group, isobutyl group, n-pentyl group, isopentyl group, neopentyl group, n-hexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, n-nonadecyl group, and n-docosyl group; a cycloalkyl group such as cyclopropyl group, cyclobutyl group, cyclopentyl group, and cyclohexyl group; an alkenyl group such as vinyl group, allyl group, 1-propenyl group, isopropenyl group, 1-butenyl group, 2-butenyl group, 2-methylallyl group, 1-hexenyl group, 1-heptenyl group, 1-octenyl group, and 2-methyl-1-propenyl group; and an alkynyl group such as propargyl group.

Examples of the unsubstituted aromatic hydrocarbon group include phenyl group, 1-naphthyl group, 2-naphthyl group, 1-phenanthryl group, 2-phenanthryl group, 3-phenanthryl group, 4-phenanthryl group, 9-phenanthryl group, 1-anthryl group, 2-anthryl group, 9-anthryl group, 1-pyrenyl group, 2-pyrenyl group, 4-pyrenyl group, 1-triphenylenyl group, 2-triphenylenyl group, 2-pyridyl group, 3-pyridyl group, and 4-pyridyl group.

When the hydrocarbon group represented by R¹ has a substituent, examples of such substituent include: deuterium atom; an alkyl group having 1 to 4 carbon atoms such as methyl group, ethyl group, n-propyl group, isopropyl group, n-butyl group, sec-butyl group, isobutyl group, and tert-butyl group; a cycloalkyl group having 3 to 6 carbon atoms such as cyclopropyl group, cyclobutyl group, cyclopentyl group, and cyclohexyl group; an aromatic hydrocarbon group having 6 to 10 carbon atoms such as phenyl group, 1-naphthyl group, and 2-naphthyl group; a halogeno group such as fluoro group, chloro group, bromo group, and iodo group; an oxygen-containing functional group such as an alkoxy group having 1 to 4 carbon atoms, carboxy group, carbonyl group, and hydroxyl group; a nitrogen-containing functional group such as cyano group; a sulfur-containing functional group such as an alkylthio group; a functional groups containing oxygen atoms and nitrogen atoms such as an amide group, an imide group, a urea group, a group containing a urethane structure, a group containing an isocyanuric structure, nitro group, nitroso group, cyanate group, isocyanate group, and morpholino group; an oxygen-containing heterocyclic group such as furanyl group; a sulfur-containing heterocyclic group such as thienyl group; and a nitrogen-containing heterocyclic group such as pyrrolyl group and pyridyl group.

When the hydrocarbon group represented by R¹ has a substituent, preferred examples of R¹ include: an alkyl-substituted phenyl group such as 2-methylphenyl group, 3-methylphenyl group, and 4-methylphenyl group; an alkoxy-substituted phenyl group such as 2-methoxyphenyl group, 3-methoxyphenyl group, and 4-methoxyphenyl group; a halogen-substituted phenyl group such as 2-chlorophenyl group, 3-chlorophenyl group, 4-chlorophenyl group, 2-bromophenyl group, 3-bromophenyl group, and 4-bromophenyl group; a nitro-substituted phenyl group such as 4-nitrophenyl group and 2-nitrophenyl group; an aromatic-substituted alkyl group such as benzyl group, phenethyl group, 1-naphthylmethyl group, and 2-naphthylmethyl group; a cycloalkyl-substituted alkyl group such as cyclohexylmethyl group; a hydrocarbon group having an oxygen-containing heterocycle such as furfuryl group; a hydrocarbon group having a sulfur-containing heterocycle such as thienylmethyl group; and a hydrocarbon group having a nitrogen-containing heterocycle such as pyridylmethyl group.

Among the hydrocarbon groups mentioned above, R¹ is preferably the aliphatic hydrocarbon group, more preferably the substituted or unsubstituted aliphatic hydrocarbon group having 1 to 24 carbon atoms, still more preferably the alkyl group having 1 to 24 carbon atoms, even more preferably the alkyl group having 1 to 12 carbon atoms, particularly preferably the alkyl group having 1 to 4 carbon atoms, and most preferably the alkyl group having 1 to 2 carbon atoms.

When m is 2, the two R¹ present in one molecule of the carbonic acid monoester salt may be identical or different groups, but are preferably identical groups.

### (A^{m+} and m)

A^{m+} is an m-valent cation derived from a base represented by A. Further, m is 1 or 2, preferably 1.

A^{m+} is not particularly limited, and is preferably one or more cations selected from the group consisting of, for example, an ammonium cation, an amidinium cation, a guanidinium cation, a phosphonium cation, a phosphazenium cation, a carbocation, a Group 1 metal cation, and a Group 2 metal cation.

Examples of the ammonium cation include: a primary ammonium cation such as methylammonium cation, ethylammonium cation, isopropylammonium cation, n-butylammonium cation, and 2-hydroxyethylammonium cation; a secondary ammonium cation such as dimethylammonium cation, diethylammonium cation, and dicyclohexylammonium cation; a tertiary ammonium cation such as trimethylammonium cation and triethylammonium cation; and a quaternary ammonium cation such as tetramethylammonium cation, tetraethylammonium cation, tetrapropylammonium cation, tetrabutylammonium cation, phenyltrimethylammonium cation, and benzyltrimethylammonium cation.

Examples of an amidinium cation include, for example, formamidinium cation which is the protonated form of formamidine; acetamidinium cation which is the protonated form of acetamidine; 1,5-diazabicyclo[4.3.0]non-5-enium cation which is the protonated form of 1,5-diazabicyclo[4.3.0]non-5-ene; 1,8-diazabicyclo[5.4.0]undec-7-enium cation which is the protonated form of 1,8-diazabicyclo[5.4.0]undec-7-ene; and derivatives obtained by introducing substituents to these. Examples of the substituents include those described as substituents that the hydrocarbon group represented by R¹ may have.

Examples of the guanidinium cation include, for example, 1,1,3,3-tetramethylguanidinium cation which is the protonated form of 1,1,3,3-tetramethylguanidine; 2-tert-butyl-1,1,3,3-tetramethylguanidinium cation which is the protonated form of 2-tert-butyl-1,1,3,3-tetramethylguanidine; 1,5,7-triazabicyclo[4.4.0]dec-5-enium cation which is the protonated form of 1,5,7-triazabicyclo[4.4.0]dec-5-ene; 7-methyl-1,5,7-triazabicyclo[4.4.0]dec-5-enium cation which is the protonated form of 7-methyl-1,5,7-triazabicyclo[4.4.0]dec-5-ene; and derivatives obtained by introducing substituents to these. Examples of the substituents include those described as substituents that the hydrocarbon group represented by R¹ may have.

Examples of the phosphonium cation include: a tertiary phosphonium cation such as triphenylphosphonium cation and tri-tert-butylphosphonium cation; and a quaternary phosphonium cation such as tetraphenylphosphonium cation, tetra-p-tolylphosphonium cation, triphenylbenzylphosphonium cation, triphenylbutyl cation, tetraethylphosphonium cation, and tetrabutylphosphonium cation.

Examples of the phosphazenium cation include, for example, tert-butylimino-tris(dimethylamino)phosphoranium cation which is the protonated form of tert-butylimino-tris(dimethylamino)phosphorane; tert-butylimino-tri(pyrrolidino)phosphoranium cation which is the protonated form of tert-butylimino-tri(pyrrolidino)phosphorane; 2-tert-butylimino-2-diethylamino-1,3-dimethylperhydro-1,3,2-diazaphospholinium cation which is the protonated form of 2-tert-butylimino-2-diethylamino-1,3-dimethylperhydro-1,3,2-diazaphospholine; 1-tert-butyl-2,2,4,4,4-pentakis(dimethylamino)-2λ⁵,4λ⁵-catenadi(phosphazenium) cation which is the protonated form of 1-tert-butyl-2,2,4,4,4-pentakis(dimethylamino)-2λ⁵,4λ⁵-catenadi(phosphazene); 1-tert-butyl-4,4,4-tris(dimethylamino)-2,2-bis[tris(dimethylamino)phosphoranylideneamino]-2λ⁵,4λ⁵-catenadi(phosphazenium) cation which is the protonated form of 1-tert-butyl-4,4,4-tris(dimethylamino)-2,2-bis[tris(dimethylamino)phosphoranylideneamino]-2λ⁵,4λ⁵-catenadi(phosphazene); and derivatives obtained by introducing substituents to these. Examples of the substituents include those described as substituents that the hydrocarbon group represented by R¹ may have.

Examples of the carbocation include a monovalent carbocation such as triphenylmethyl cation, tropylium cation, and azulenium cation.

Examples of the Group 1 metal cation include lithium cation, sodium cation, and potassium cation.

Examples of the Group 2 metal cation include magnesium cation and calcium cation.

From the viewpoint of the availability of A and the yield of the carbonic acid diester, A^{m+} is preferably a cation selected from the group consisting of the ammonium cation, the amidinium cation, the guanidinium cation, the phosphonium cation, the phosphazenium cation, and the carbocation, more preferably a cation selected from the group consisting of the ammonium cation, the amidinium cation, the guanidinium cation, the phosphonium cation, and the phosphazenium cation, still more preferably a cation selected from the group consisting of the amidinium cation and the guanidinium cation, and particularly preferably the amidinium cation.

Specific examples of compounds represented by general formula (1) include compounds represented by the formula below, with counter cations being the amidinium cation, the guanidinium cation, the phosphonium cation, the phosphazenium cation, the carbocation, the Group 1 metal cation, or the Group 2 metal cation, other than 1,8-diazabicyclo[5.4.0]undec-7-enium cation. Such compounds include amidinium salts, guanidinium salts, phosphonium salts, phosphazenium salts, carbocation salts, Group 1 metal salts, and Group 2 metal salts.

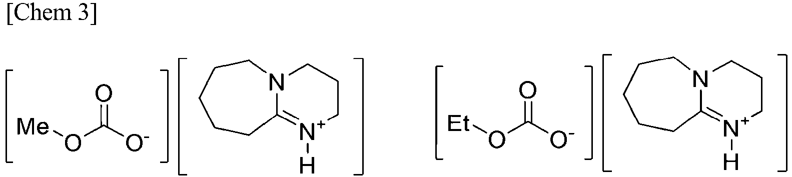

The carbonic acid monoester salt may be a commercially available product, or may be produced by a known production method or a method analogous thereto, or may be produced by the carbonic acid monoester salt production step described later. In the carbonic acid monoester salt production step described later, it is possible to produce the carbonic acid monoester salt without using phosgene and sacrificial reagents, therefore, the carbonic acid monoester salt is preferably produced by such a step.

The amount of the carbonic acid monoester salt (charged amount; total amount if multiple types are used) in the reaction step is not particularly limited, but in the present embodiment, since it is considered that two molecules of the alkoxysilane react with one molecule of the carbonic acid monoester salt to produce the carbonic acid diester, it is preferably 0.1 equivalent or more and 0.5 equivalent or less relative to the alkoxysilane.

In the present disclosure, "equivalent" means chemical equivalent (molar equivalent).

### 1-2. Alkoxysilane

The alkoxysilane is not particularly limited and can be appropriately selected according to the target carbonic acid diester. In the present disclosure, alkoxysilane means alkoxysilanes and their derivatives thereof. Further, alkoxysilanes may be used alone, or two or more types may be used in combination in any combination and ratio.

In the present embodiment, the alkoxysilane is preferably a compound represented by general formula (2).

Si(OR¹)ₚ(R²)₄₋ₚ (2)

### (R¹)

R¹ is a substituted or unsubstituted hydrocarbon group. R¹ in general formula (2) has the same meaning as R¹ in general formula (1), and its preferred embodiments are also the same.

When p is from 2 to 4, the plurality of R¹ groups may be the same or different.

Further, R¹ in general formula (2) may be the same as or different from R¹ in general formula (1), but from the viewpoint of efficiently producing a single carbonic acid diester, it is preferably the same group.

### (R²)

R² is a substituted or unsubstituted hydrocarbon group. R² has the same meaning as the substituted or unsubstituted hydrocarbon group represented by R¹.

When p is 1 or 2, the plurality of R² groups may be the same or different.

From the viewpoint of the availability and stability of the alkoxysilane, R² is preferably a methyl group, an ethyl group, a vinyl group, an allyl group, or a phenyl group.

When the hydrocarbon group represented by R² has a substituent, preferred substituents include isocyanate group and cyano group.

### (p)

p is an integer from 1 to 4, preferably an integer from 2 to 4, more preferably an integer from 3 to 4, and still more preferably 4, from the viewpoint of reaction efficiency.

That is, the alkoxysilane represented by general formula (2) is a compound selected from the group consisting of a monoalkoxysilane, a dialkoxysilane, a trialkoxysilane, and a tetraalkoxysilane; preferably a compound selected from the group consisting of the dialkoxysilane, the trialkoxysilane, and the tetraalkoxysilane; more preferably a compound selected from the group consisting of the trialkoxysilane and the tetraalkoxysilane; and still more preferably the tetraalkoxysilane.

Specific examples of the monoalkoxysilane include methoxytrimethylsilane, methoxytriethylsilane, methoxytripropylsilane, methoxytriisobutylsilane, methoxytrioctylsilane, methoxytrimethoxyhexadecylsilane, methoxytrivinylsilane, methoxytriphenylsilane, phenylmethoxydimethylsilane, phenylmethoxydiethylsilane, ethoxytrimethylsilane, ethoxytriethylsilane, ethoxytripropylsilane, ethoxytriisobutylsilane, ethoxytrioctylsilane, ethoxytriphenylsilane, ethoxytrivinylsilane, ethoxytriallylsilane, ethoxydiethylphenylsilane, phenylethoxydipropylsilane, propoxytrimethylsilane, propoxytriethylsilane, propoxytripropylsilane, phenylpropoxydimethylsilane, phenylpropoxydiethylsilane, and phenylpropoxydipropylsilane. Among these, from the viewpoint of availability and reactivity, the monoalkoxysilane is preferably ethoxytrimethylsilane.

Specific examples of the dialkoxysilane include dimethoxydimethylsilane, dimethoxydiethylsilane, dimethoxydipropylsilane, phenylmethoxydimethylsilane, dimethoxymethylvinylsilane, dimethoxydiphenylsilane, diethoxydimethylsilane, diethoxydiethylsilane, diethoxydipropylsilane, diethoxymethylphenylsilane, diethoxyethylphenylsilane, diethoxyphenylpropylsilane, dipropoxydimethylsilane, dipropoxydiethylsilane, dipropoxydipropylsilane, phenyl(dipropoxy)methylsilane, phenyl(dipropoxy)ethylsilane, phenyl(dipropoxy)propylsilane, dibutoxydimethylsilane, dibutoxydiethylsilane, and phenyldimethoxyethylsilane. Among these, from the viewpoint of availability and reactivity, the dialkoxysilane is preferably a compound selected from the group consisting of dimethoxydimethylsilane and diethoxydimethylsilane.

Specific examples of the trialkoxysilane include trimethoxymethylsilane, trimethoxyethylsilane, trimethoxypropylsilane, trimethoxyisobutylsilane, trimethoxyoctylsilane, trimethoxyhexadecylsilane, triethoxymethylsilane, triethoxyethylsilane, triethoxypropylsilane, triethoxyisobutylsilane, triethoxyoctylsilane, trimethoxyvinylsilane, trimethoxyphenylsilane, triethoxyphenylsilane, triethoxyvinylsilane, triethoxyallylsilane, tripropoxymethylsilane, tripropoxyethylsilane, tripropoxypropylsilane, tripropoxyphenylsilane, 2-cyanoethyltriethoxysilane, and (3-isocyanatopropyl)triethoxysilane. Among these, from the viewpoint of availability and reactivity, the trialkoxysilane is preferably a compound selected from the group consisting of trimethoxymethylsilane, triethoxymethylsilane, triethoxyphenylsilane, triethoxyvinylsilane, and triethoxyallylsilane.

Specific examples of the tetraalkoxysilane include tetramethoxysilane, tetraethoxysilane, tetrapropoxysilane, tetraisopropoxysilane, tetrabutoxysilane, and tetrakis(2-ethylhexyloxy)silane. Among these, from the viewpoint of availability and reactivity, the tetraalkoxysilane is preferably a compound selected from the group consisting of tetramethoxysilane, tetraethoxysilane, tetrapropoxysilane, and tetrabutoxysilane, and more preferably tetraethoxysilane.

### 1-3. Catalyst

In the reaction step, one or more catalysts selected from the group consisting of a cerium catalyst and a tin catalyst are used as a catalyst for the reaction between the carbonic acid monoester salt and the alkoxysilane.

The cerium catalyst is not particularly limited, and examples include cerium(IV) oxide, cerium(IV) sulfate, cerium(IV) fluoride, cerium(IV) trifluoromethanesulfonate, cerium(III) fluoride, cerium(III) chloride, cerium(III) bromide, cerium(III) iodide, cerium(III) acetate, cerium stearate, cerium carbonate, cerium(III) trifluoromethanesulfonate, cerium(III) tungstate, cerium(III) oxalate, and cerium(III) phosphate. Preferably, it is one or more selected from the group consisting of cerium(IV) oxide, cerium(IV) sulfate, cerium(IV) fluoride, and cerium(IV) trifluoromethanesulfonate, and particularly preferably cerium(IV) oxide.

The tin catalyst is not particularly limited, and examples include a dialkyltin bis(aliphatic monocarbonate) such as dimethyltin dilaurate, dibutyltin dilaurate, dibutyltin maleate, dibutyltin diacetate, and dioctyltin dilaurate; a dialkyltin oxide such as dibutyltin oxide and dioctyltin oxide; and a dialkyltin dihalide such as dibutyltin dichloride and dibutyltin dibromide. Preferably, the tin catalyst is one or more selected from the group consisting of the dialkyltin bis(aliphatic monocarbonate) and the dialkyltin oxide.

Among these, from the viewpoint of catalytic activity, the catalyst is preferably one or more selected from the group consisting of cerium(IV) oxide, the dialkyltin bis(aliphatic monocarbonate), and the dialkyltin oxide, more preferably one or more selected from the group consisting of cerium(IV) oxide, dibutyltin dilaurate, and dibutyltin oxide, and still more preferably cerium(IV) oxide.

The amount of the catalyst (charged amount; total amount if multiple types are used) in the reaction step may be appropriately selected according to the type of the carbonic acid monoester salt, the type of the alkoxysilane, the type of the catalyst, and so on. Specifically, the amount of the catalyst used relative to the alkoxysilane is preferably 0.5 mol% or more, more preferably 1.0 mol% or more, still more preferably 5.0 mol% or more, and particularly preferably 10.0 mol% or more. Further, it is usually 70.0 mol% or less, preferably 50.0 mol% or less, more preferably 40.0 mol% or less, and still more preferably 30.0 mol% or less. That is, suitable ranges for the amount of the catalyst used include, for example, 0.5 mol% or more and 70.0 mol% or less, 1.0 mol% or more and 50.0 mol% or less, 5.0 mol% or more and 40.0 mol% or less, and 10.0 mol% or more and 30.0 mol% or less.

### 1-4. Reaction Mechanism

The present inventors infer that the reaction mechanism for producing the carbonic acid diester by the production method according to this embodiment is as shown in the scheme below. The following scheme is an example where the compound represented by general formula (1) with m being 1 in the formula is used as the carbonic acid monoester, and the compound represented by general formula (2) with p being 4 in the formula (i.e., a tetraalkoxysilane) is used as the alkoxysilane. As shown in the scheme below, it is inferred that in the production method according to this embodiment, one molecule of the carbonic acid monoester salt and two molecules of the alkoxysilane are consumed to produce one molecule of the carbonic acid diester. Furthermore, in the scheme below, it is considered that an exchange occurs between the OR¹ group of the carbonic acid monoester and the OR¹ group of the alkoxysilane, potentially yielding the carbonic acid diester having two OR¹ groups derived from the carbonic acid monoester, the carbonic acid diester having one OR¹ group derived from the carbonic acid monoester and one OR¹ group derived from the alkoxysilane, and the carbonic acid diester having two OR¹ groups derived from the alkoxysilane.

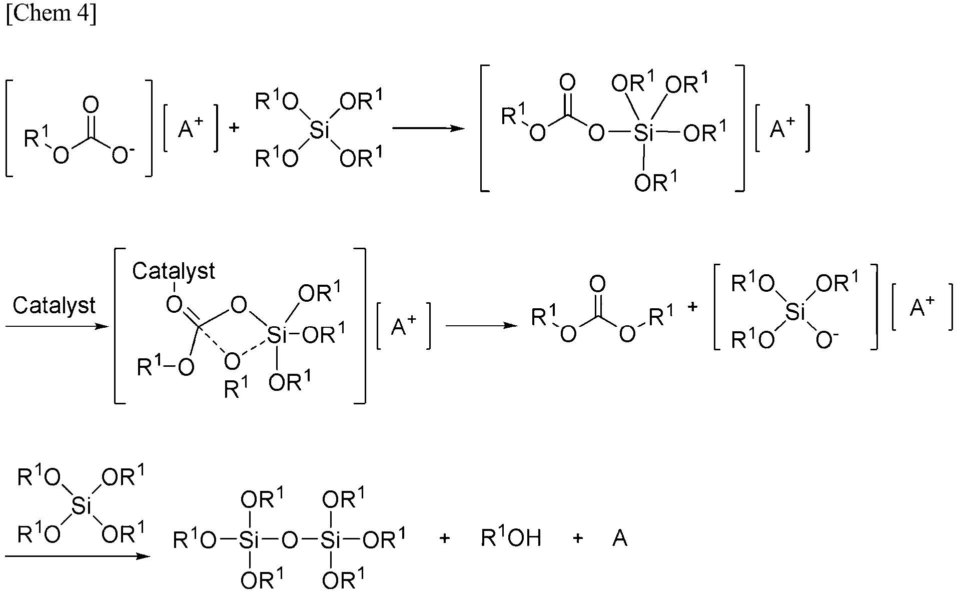

### 1-5. Carbonic Acid Diester

The carbonic acid diester produced by the production method according to this embodiment is a carbonic acid diester having two ester groups selected from the group consisting of an ester group derived from the carbonic acid monoester salt and an ester group derived from an alkoxy group of the alkoxysilane. The carbonic acid diester produced by the production method according to this embodiment is not particularly limited and may be determined according to the intended use of the carbonic acid diester. Examples of the intended use of the carbonic acid diester include solvents for electrolytes, alkylating agents, carbonylating agents, additives for gasoline, additives for diesel fuel, and raw materials for polymers such as polyurethanes and polycarbonates.

In the reaction step of this embodiment, it is preferred to use the compound represented by general formula (1) as the carbonic acid monoester salt, and it is preferred to use the compound represented by general formula (2) as the alkoxysilane. Therefore, in the production method according to this embodiment, a carbonic acid diester represented by general formula (3) is suitably produced, as shown in the reaction scheme below.

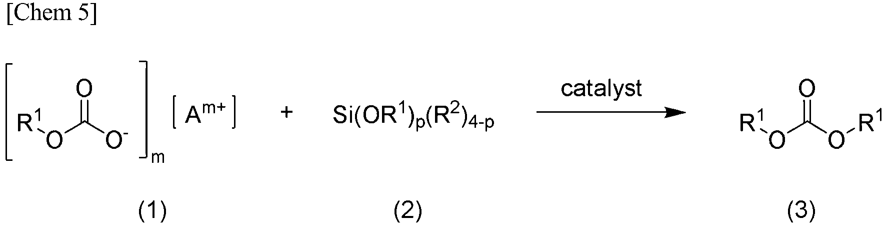

In general formulae (1) to (3), R¹, R², A^{m+}, m, and p are as described above. In general formula (3), the two R¹ groups may be the same or different, but are preferably the same group.

Specific examples of the carbonic acid diester represented by general formula (3) include dimethyl carbonate, diethyl carbonate, di-n-propyl carbonate, diisopropyl carbonate, di-n-butyl carbonate, ethyl methyl carbonate, ethyl isopropyl carbonate, dicyclohexyl carbonate, divinyl carbonate, diallyl carbonate, methyl propargyl carbonate, and diphenyl carbonate.

### 1-6. Reaction Solvent

In the reaction step, the reaction between the carbonic acid monoester salt and the alkoxysilane may be carried out in a solvent or without a solvent. From the viewpoint of achieving the formation of the carbonic acid monoester salt under milder conditions, it is preferable to carry out the reaction under solvent-free conditions. In other words, carrying out the reaction under solvent-free conditions increases the opportunities for contact between the carbonic acid monoester salt and the alkoxysilane, which has the advantages of improving the reaction rate, enabling a shorter reaction time, or allowing catalytic reactions at lower reaction temperatures, and is therefore preferred. Furthermore, if the solubility of the produced carbonic acid diester in the reaction solution is low, it is also preferable to add a good solvent such as an aprotic polar solvent to the reaction system. This is because adding a good solvent to the reaction solution is thought to facilitate the dissolution of the carbonic acid diester in the reaction solution, allowing the reaction to proceed smoothly.

In this disclosure, "solvent-free conditions" means not using a reaction solvent different from the reaction reagents. For example, if a reaction substrate such as an alcohol is also used as a reaction solvent, that reaction substrate is not considered a reaction solvent and falls under solvent-free conditions.

When the reaction step is carried out in a reaction solvent, the reaction solvent may be used alone, or two or more types may be used in any combination and ratio.

The reaction solvent is not particularly limited as long as it does not inhibit the effects of the present disclosure, and examples include an aliphatic hydrocarbon such as butane, hexane, octane, and cyclohexane; an aromatic hydrocarbon such as benzene, toluene, and xylene; a heterocyclic aromatic compound such as pyridine; a aprotic polar solvent such as N,N-dimethylformamide, dimethylacetamide, dimethyl sulfoxide, 1,3-dimethyl-2-imidazolidinone, and N-methylpyrrolidone (NMP); an ether such as diethyl ether, diisopropyl ether, 1,2-dimethoxyethane, tetrahydrofuran, and dioxane; a nitrile such as acetonitrile, propionitrile, butyronitrile, benzonitrile, and 2-cyanopyridine; and a ketone such as acetone, isopropyl methyl ketone, and methyl isobutyl ketone. Among these, from the viewpoint of high solubility of the carbonic acid diester, the reaction solvent is preferably the aprotic polar solvent, and more preferably N-methylpyrrolidone (NMP).

When the reaction step is carried out in the presence of the reaction solvent, the amount of reaction solvent (charged amount; total amount if multiple types are used) is not particularly limited as long as it does not inhibit the effects of the present disclosure. Specifically, the amount of the reaction solvent per 1.0 mmol of the carbonic acid monoester salt is usually 0.5 mL or more, preferably 1.0 mL or more, and more preferably 2.0 mL or more. It is also usually 10.0 mL or less, and preferably 5.0 mL or less. That is, suitable ranges for the amount of the reaction solvent used include, for example, 0.5 mL to 10.0 mL, 1.0 mL to 10.0 mL, and 2.0 mL to 5.0 mL per 1.0 mmol of the carbonic acid monoester salt.

By setting the amount of reaction solvent within the above range, opportunities for contact between the carbonic acid monoester salt and the alkoxysilane can be ensured. Furthermore, if the reaction solvent is a good solvent for the reaction substrate and product, it can improve the solubility of the reaction substrate and product, allowing the reaction to proceed smoothly.

### 1-7. Reaction Temperature

The reaction temperature in the reaction step is not particularly limited and may be appropriately adjusted according to the type of the reaction substrate, the type of the catalyst, the presence or absence of a solvent, and so on. Specifically, the reaction temperature is usually 80°C or more, preferably 100°C or more, more preferably 120°C or more, and still more preferably 130°C or more. It is also usually 250°C or less, preferably 200°C or less, more preferably 160°C or less, and still more preferably 150°C or less. That is, suitable ranges for the reaction temperature include 80°C or more and 250°C or less, 100°C or more and 200°C or less, 120°C or more and 160°C or less, and 130°C or more and 150°C or less.

According to the production method of this embodiment, since a high reaction rate can be achieved by selecting an appropriate catalyst, it is possible to produce the carbonic acid diester under milder temperature conditions.

### 1-8. Reaction Time

The reaction time in the reaction step is not particularly limited and may be appropriately adjusted depending on the reaction temperature, catalyst amount, reaction scale, and so on. Specifically, the reaction time is usually 1 hour or more, preferably 3 hours or more, more preferably 5 hours or more, and still more preferably 10 hours or more. It is also usually 120 hours or less, preferably 100 hours or less, more preferably 80 hours or less, and still more preferably 60 hours or less. That is, suitable ranges for the reaction time include 1 hour or more and 120 hours or less, 3 hours or more and 100 hours or less, 5 hours or more and 80 hours or less, and 10 hours or more and 60 hours or less. Thus, according to the production method of this embodiment, since a high reaction rate can be achieved by selecting an appropriate catalyst, it is possible to produce the carbonic acid diester in a relatively short reaction time.

### 1-9. Operation Procedure

The reaction step can be carried out, for example, as follows.

First, the carbonic acid monoester salt, alkoxysilane, catalyst, and, if necessary, a reaction solvent are fed to a reactor. This operation may be performed under an air atmosphere or an inert gas atmosphere such as nitrogen or argon. The reactor is not particularly limited as long as it is formed from a material stable to the reaction reagents and the carbonic acid diester. If the production method according to this embodiment includes the carbonic acid monoester salt production step described later, the reactor used in the carbonic acid monoester salt production step may be used as is for the reaction step.

Next, by heating the reaction solution, the reaction between the carbonic acid monoester salt and the alkoxysilane is carried out. During the reaction, it is preferable to stir the reaction solution. The method of stirring the reaction solution is not particularly limited, and for example, a method of stirring the reaction solution by stirring means such as a magnetic stirrer or an impeller can be adopted. In addition to stirring, a flow-type reactor can be adopted, in which the reaction solution is continuously supplied to a reaction tube equipped with a catalyst and heated.

Alternatively, if the production method according to this embodiment includes the carbonic acid monoester salt production step described later, the reaction step may be carried out as follows.

First, alcohol, a base, alkoxysilane, and a catalyst are added to the reactor.

Next, a carbon dioxide-containing gas is supplied into the reactor to react the alcohol, base, and carbon dioxide to produce the carbonic acid monoester salt.

Subsequently, the reaction step is carried out in this reactor without purifying the carbonic acid monoester salt. That is, the reaction solution is heated in this reactor, and the carbonic acid monoester salt and the alkoxysilane are reacted to produce the carbonic acid diester.

Since the alcohol and base used in the carbonic acid monoester salt production step do not inhibit the reaction in the reaction step, the reaction step can be carried out in the presence of either or both of the alcohol and the base. Furthermore, since neither the alkoxysilane nor the catalyst used in the reaction step inhibits the carbonic acid monoester salt production step, the carbonic acid monoester salt production step can be carried out in the presence of either or both of the alkoxysilane and the catalyst. Moreover, the reaction in the carbonic acid monoester salt production step proceeds at a lower temperature than the reaction in the subsequent reaction step. Therefore, as described above, by having the alkoxysilane and catalyst present in the reaction system from the beginning of the reaction when producing the carbonic acid monoester salt, the carbonic acid monoester salt production step and the reaction step can be carried out continuously without adding the alkoxysilane and catalyst to the reactor after the carbonic acid monoester salt production step.

### 2. Carbonic Acid Monoester Salt Production Step

In this embodiment, the carbonic acid monoester salt may be a commercially available product or may be synthesized and used; however, it is preferable to synthesize and use the carbonic acid monoester salt.

When synthesizing the carbonic acid monoester salt, specifically, a carbonic acid monoester salt production step may be carried out before the reaction step, in which an alcohol, a base, and a carbon dioxide-containing gas are brought into contact. At this time, it is preferable to set the ratio of the partial pressure P_{CO2} of carbon dioxide gas in the carbon dioxide-containing gas to the total pressure Pₜ of the carbon dioxide-containing gas to 0.0001 or more and 1.00 or less.

2-1. Alcohol

The alcohol is an alcohol having a hydrocarbon group from which the carbonic acid monoester salt is derived, and is selected according to the target carbonic acid monoester salt to be synthesized.

When the carbonic acid monoester salt synthesized in the carbonic acid monoester salt production step is a compound represented by general formula (1), the alcohol is a compound represented by the following general formula (1'). In general formula (1'), R¹ has the same meaning as R¹ in general formula (1), and preferred embodiments are also the same.

R¹OH (1')

Examples of alcohols include an aliphatic alcohol and an aromatic alcohol.

Specific examples of the aliphatic alcohol include, for example, an alkyl alcohol such as methanol, ethanol, n-propyl alcohol, isopropyl alcohol, n-butyl alcohol, sec-butyl alcohol, tert-butyl alcohol, isobutyl alcohol, n-pentyl alcohol, isopentyl alcohol, neopentyl alcohol, n-hexyl alcohol, n-heptyl alcohol, n-octyl alcohol, n-nonyl alcohol, n-decyl alcohol, n-undecyl alcohol, n-dodecyl alcohol, n-tridecyl alcohol, n-tetradecyl alcohol, n-pentadecyl alcohol, n-hexadecyl alcohol, n-heptadecyl alcohol, n-octadecyl alcohol, n-nonadecyl alcohol, and n-docosyl alcohol; a cycloalkanol such as cyclopropanol, cyclobutanol, cyclopentanol, and cyclohexanol; an alkenyl alcohol such as vinyl alcohol, allyl alcohol, 1-propen-1-ol, isopropenyl alcohol, 1-buten-1-ol, 2-buten-1-ol, 2-methylallyl alcohol, 1-hexen-1-ol, 1-hepten-1-ol, 1-octen-1-ol, and 2-methyl-1-propen-1-ol; and an alkynyl alcohol such as propargyl alcohol.

Specific examples of the aromatic alcohol include phenol, 1-naphthol, 2-naphthol, 1-hydroxyphenanthrene, 2-hydroxyphenanthrene, 3-hydroxyphenanthrene, 4-hydroxyphenanthrene, 9-hydroxyphenanthrene, 1-hydroxyanthracene, 2-hydroxyanthracene, 9-hydroxyanthracene, 1-hydroxypyrene, 2-hydroxypyrene, 4-hydroxypyrene, 1-hydroxytriphenylene, 2-hydroxytriphenylene, 2-hydroxypyridine, 3-hydroxypyridine, and 4-hydroxypyridine.

The lower limit of the amount of the alcohol (charged amount), from the viewpoint of improving reaction rate, is preferably m × 1.0 equivalents or more relative to 1.0 equivalent of the amount of base (charged amount) (where m is synonymous with m in general formula (1)), more preferably m × 1.3 equivalents or more, and still more preferably m × 1.5 equivalents or more. The upper limit of the amount of alcohol (charged amount) is preferably m × 5.0 equivalents or less. That is, suitable ranges for the amount of alcohol (charged amount) include, for example, a range of m × 1.0 equivalents or more and m × 5.0 equivalents or less, a range of m × 1.3 equivalents or more and m × 5.0 equivalents or less, and a range of m × 1.5 equivalents or more and m × 5.0 equivalents or less, relative to 1.0 equivalent of the amount of base (charged amount).

### 2-2. Base

The base is a base from which the cation component forming the carbonic acid monoester salt is derived, and is selected according to the carbonic acid monoester salt to be synthesized.

When the carbonic acid monoester salt synthesized in the carbonic acid monoester salt production step is a compound represented by general formula (1), the base is a compound represented by A, from which A^{m+} is derived. That is, A is a base from which one or more cations selected from the group consisting of the ammonium cation, the amidinium cation, the guanidinium cation, the phosphonium cation, the phosphazenium cation, the carbocation, the Group 1 metal cation, and the Group 2 metal cation are derived.

Examples of the base from which the ammonium cation is derived include: a primary amine such as methylamine, ethylamine, isopropylamine, n-butylamine, and 2-hydroxyethylamine; a secondary amine such as dimethylamine, diethylamine, and dicyclohexylamine; a tertiary amine such as trimethylamine and triethylamine; and a quaternary ammonium hydroxide such as tetramethylammonium hydroxide, tetraethylammonium hydroxide, tetrapropylammonium hydroxide, tetrabutylammonium hydroxide, phenyltrimethylammonium hydroxide, benzyltrimethylammonium hydroxide, and tetrabutylammonium fluoride.

Examples of the base from which the amidinium cation is derived include formamidine, acetamidine, 1,5-diazabicyclo[4.3.0]non-5-ene, 1,8-diazabicyclo[5.4.0]undeca-7-ene, and derivatives thereof into which substituents have been introduced. Examples of the substituents include those described as substituents that the hydrocarbon group represented by R¹ in general formula (1) may have.

Examples of the base from which the guanidinium cation is derived include 1,1,3,3-tetramethylguanidine, 2-tert-butyl-1,1,3,3-tetramethylguanidine, 1,5,7-triazabicyclo[4.4.0]deca-5-ene, 7-methyl-1,5,7-triazabicyclo[4.4.0]deca-5-ene, and derivatives thereof into which substituents have been introduced. Examples of the substituents include those described as substituents that the hydrocarbon group represented by R¹ in general formula (1) may have.

Examples of the base from which the phosphonium cation is derived include: a tertiary phosphine such as triphenylphosphine and tri-tert-butylphosphine; and a quaternary phosphonium cation such as tetraphenylphosphine, tetra-p-tolylphosphine, triphenylbenzylphosphine, triphenylbutylphosphine, tetraethylphosphine, and tetrabutylphosphine.

Examples of the bases from which the phosphazenium cation is derived include tert-butylimino-tris(dimethylamino)phosphorane, tert-butylimino-tri(pyrrolidino)phosphorane, 2-tert-butylimino-2-diethylamino-1,3-dimethylperhydro-1,3,2-diazaphospholine, 1-tert-butyl-2,2,4,4,4-pentakis(dimethylamino)-2λ⁵,4λ⁵-catenadi(phosphazene), 1-tert-butyl-4,4,4-tris(dimethylamino)-2,2-bis[tris(dimethylamino)phospholanilideneamino]-2λ⁵,4λ⁵-catenadi(phosphazene), and derivatives thereof into which substituents have been introduced. Examples of the substituents include those described as substituents that the hydrocarbon group represented by R¹ in general formula (1) may have.

Examples of the bases from which the carbocation is derived include triphenylmethyl chloride, triphenylmethanol, 1,3,5-cycloheptatriene, and azulene.

Examples of the base from which the Group 1 metal cation is derived include: a Group 1 metal hydroxide such as lithium hydroxide, sodium hydroxide, and potassium hydroxide; a Group 1 metal bicarbonate such as lithium bicarbonate, sodium bicarbonate, and potassium bicarbonate; a Group 1 metal carbonate such as lithium carbonate, sodium carbonate, and potassium carbonate; and a Group 1 metal alkoxide such as lithium methoxide, sodium methoxide, potassium methoxide, lithium ethoxide, sodium ethoxide, and potassium ethoxide.

Examples of the base from which the Group 2 metal cation is derived include a Group 2 metal hydroxide such as magnesium hydroxide and calcium hydroxide.

### 2-3. Carbon Dioxide-Containing Gas

In the carbonic acid monoester salt production step, a carbon dioxide-containing gas containing carbon dioxide (gas) as a raw material is used.

The carbon dioxide may be one prepared as an industrial gas, or may be one separated and collected from exhaust gas from factories, power plants, etc. The carbon dioxide may be used alone, or may be used in the form of a mixed gas combined with gases other than carbon dioxide to the extent that the effects of the present disclosure are not significantly impaired. However, from the viewpoint of improving reactivity, it is preferable to use carbon dioxide alone. Examples of gases other than carbon dioxide mentioned above include inert gases such as nitrogen and argon.

The method for introducing the carbon dioxide-containing gas into the reaction system is not particularly limited, and may be a method of replacing the atmosphere in the reactor with carbon dioxide, or may be a method of supplying carbon dioxide into the reaction solution by bubbling. Among these, the supply method by bubbling is preferable because it can increase the contact opportunities between alcohol, base, and carbon dioxide, thereby improving reaction efficiency.

The total pressure Pₜ of the carbon dioxide-containing gas is not particularly limited, but from the viewpoint of reaction efficiency, it is preferably 0.01 MPa or more, more preferably 0.05 MPa or more, and still more preferably 0.08 MPa or more. It is also preferably 5.0 MPa or less, more preferably 3.0 MPa or less, still more preferably 1.0 MPa or less, particularly preferably 0.50 MPa or less, and most preferably 0.20 MPa or less. That is, suitable ranges for the total pressure Pₜ of the carbon dioxide-containing gas include, for example, a range of 0.01 MPa or more and 5.0 MPa or less, 0.01 MPa or more and 3.0 MPa or less, 0.05 MPa or more and 1.0 MPa or less, 0.05 MPa or more and 0.50 MPa or less, and 0.08 MPa or more and 0.20 MPa or less. When carbon dioxide is introduced into the reaction system by bubbling, the total pressure Pₜ of the carbon dioxide-containing gas is preferably approximately the same as atmospheric pressure, i.e., (0.1 ± 0.05) MPa, from the viewpoint of work efficiency.

The partial pressure P_{CO2} of carbon dioxide gas in the carbon dioxide-containing gas is not particularly limited, but from the viewpoint of reaction efficiency, it is preferably 0.005 MPa or more, more preferably 0.01 MPa or more, still more preferably 0.05 MPa or more, and particularly preferably 0.10 MPa or more. It is also preferably 5.0 MPa or less, more preferably 3.0 MPa or less, still more preferably 1.0 MPa or less, particularly preferably 0.50 MPa or less, and most preferably 0.20 MPa or less. That is, suitable ranges for the partial pressure P_{CO2} of carbon dioxide gas in the carbon dioxide-containing gas include, for example, a range of 0.005 MPa or more and 5.0 MPa or less, 0.01 MPa or more and 3.0 MPa or less, 0.05 MPa or more and 1.0 MPa or less, 0.10 MPa or more and 0.50 MPa or less, and 0.005 MPa or more and 0.20 MPa or less.

In the carbonic acid monoester salt production step, the ratio (P_{CO2}/Pₜ) of the partial pressure P_{CO2} of carbon dioxide gas in the carbon dioxide-containing gas to the total pressure Pₜ of the carbon dioxide-containing gas is not particularly limited, but is preferably 0.05 or more, more preferably 0.10 or more, and still more preferably 0.50 or more. It is also usually 1.00 or less, preferably 0.80 or less, more preferably 0.60 or less, still more preferably 0.50 or less, and particularly preferably 0.30 or less. That is, suitable ranges for P_{CO2}/Pₜ in the carbonic acid monoester salt production step include, for example, a range of 0.05 or more and 1.00 or less, 0.10 or more and 0.80 or less, 0.50 or more and 0.60 or less, 0.05 or more and 0.50 or less, and 0.05 or more and 0.30 or less.

Note that the above-mentioned pressure means absolute pressure. Also, the "total pressure Pₜ of the carbon dioxide-containing gas" and "partial pressure P_{CO2} of carbon dioxide gas in the carbon dioxide-containing gas" refer to the pressure (at 25°C) immediately after introducing the carbon dioxide-containing gas into the reactor, i.e., at the start of the reaction.

### 2-4. Reaction Solvent

In the carbonic acid monoester salt production step, the reaction among the alcohol, the base, and carbon dioxide may be carried out in a solvent or solvent-free. From the viewpoint of achieving the production of the carbonic acid monoester salt under milder conditions, for example, from the viewpoint of improving the reaction rate and shortening the reaction time, it is preferable to carry out the reaction under solvent-free conditions.

The type of reaction solvent is not particularly limited and can be appropriately selected from a nonpolar solvent, a protic polar solvent, and an aprotic polar solvent, and is preferably one or more selected from the group consisting of the protic polar solvent and the aprotic polar solvent. The reaction solvent may be used alone or in combination of two or more types in any combination and ratio.

Examples of the nonpolar solvent include, for example, an aliphatic hydrocarbon such as butane, hexane, octane, and cyclohexane; an aromatic hydrocarbon such as benzene, toluene, and xylene; and an ether such as 1,4-dioxane and diethyl ether.

Examples of the protic polar solvent include, for example, a carboxylic acid such as formic acid and acetic acid.

Examples of the aprotic polar solvent include, for example, a tertiary carboxylic acid amide; a sulfoxide such as dimethyl sulfoxide; a ketone such as acetone and isopropyl ketone; a lactone such as γ-butyrolactone; a lactam such as N-methylpyrrolidone (NMP); a nitrile such as acetonitrile, propionitrile, butyronitrile, benzonitrile, and 2-cyanopyridine; a urea derivative; a sulfone; a carboxylic acid ester such as ethyl acetate; and a carbonic acid ester. Among these, from the viewpoints of improving reaction rate, ease of availability, and cost, the aprotic polar solvent is preferably N-methylpyrrolidone.

The amount of the reaction solvent used is not particularly limited, but is usually 0.5 times or more, preferably 1.0 times or more, and more preferably 1.5 times or more, relative to the volume of the base. It is also usually 20 times or less, preferably 15 times or less, and more preferably 10 times or less, relative to the volume of the base

### 2-5. Reaction Temperature

The reaction temperature in the carbonic acid monoester salt production step is not particularly limited, and is usually 1°C or more and 50°C or less. From the viewpoint of economic efficiency, it is preferably room temperature (r.t.). In the present disclosure, room temperature refers to a temperature range of 15°C or more and 30°C or less.

### 2-6. Reaction Time

The reaction time for the alcohol, base, and carbon dioxide is not particularly limited, and may be appropriately adjusted depending on the reaction temperature, reaction scale, and the like. Specifically, the reaction time is preferably 5 minutes or more, more preferably 10 minutes or more, and still more preferably 20 minutes or more, and is usually 48 hours or less, preferably 24 hours or less, more preferably 20 hours or less, and still more preferably 3 hours or less. That is, suitable ranges for the reaction time include, for example, 5 minutes or more and 48 hours or less, 5 minutes or more and 24 hours or less, 10 minutes or more and 20 hours or less, and 20 minutes or more and 3 hours or less.

In the carbonic acid monoester salt production step, "reaction time" refers to the carbon dioxide-containing gas supply time during which the carbon dioxide-containing gas is continuously supplied into the reactor.

### 2-7. Reactor

The reactor used in the carbonic acid monoester salt production step is not particularly limited as long as it is formed from a material stable to the reaction reagents and the carbonic acid monoester salt, and may be selected depending on the method of introducing the carbon dioxide-containing gas into the reaction system.

When the carbon dioxide-containing gas is introduced into the reaction system by replacing the atmosphere inside the reactor, the reactor is preferably a sealed reactor (closed reactor), more preferably a sealed pressure-resistant reactor, and still more preferably a stainless steel autoclave. Furthermore, the reactor preferably has a volume 10 times or more and 100 times or less the volume of the mixture containing the alcohol, the base, and optionally the reaction solvent.

When carbon dioxide is introduced into the reaction system by bubbling, the reactor preferably has a supply tube for supplying carbon dioxide to the reaction system by bubbling, and an exhaust tube for discharging gas from inside the reactor. Furthermore, the reactor preferably has a volume 1.5 times or more and 100 times or less the volume of the mixture containing the alcohol, the base, and optionally the reaction solvent.

The reactor may be equipped with a magnetic stirrer or stirring blades for stirring the reaction solution.

### 2-8. Operation Procedure

The carbonic acid monoester salt production step can be carried out, for example, as follows.

First, the raw materials, alcohol and base, are added to the reactor. At this time, it is preferable to purge the reactor with an inert gas atmosphere, such as nitrogen or argon. Also, at this time, as described in "1-8. Operation Procedure " above, an alkoxysilane and a catalyst to be used in the reaction step may be added to the reactor.

Next, a carbon dioxide-containing gas is supplied into the reactor to react the alcohol, base, and carbon dioxide. If a solvent is used, the solvent may be added to the reactor before introducing carbon dioxide, for example, simultaneously with the alcohol. During the reaction, it is preferable to stir the reaction solution with a magnetic stirrer or stirring blades.

After the reaction, the reaction solution is cooled, residual gas is discharged, and then the reaction product is collected.

### 2-9. Others

After the carbonic acid monoester salt production step, the obtained carbonic acid monoester salt can be purified by purification methods commonly performed in the field of organic synthesis, such as filtration, adsorption, column chromatography, and distillation, before being subjected to the subsequent reaction step.

Alternatively, the reaction mixture containing the carbonic acid monoester salt obtained in the carbonic acid monoester salt production step may be subjected to the reaction step without purification. Since the alcohol and base used in the carbonic acid monoester salt production step do not inhibit the reaction in the subsequent reaction step, the reaction mixture containing these compounds will not adversely affect the progress of the reaction even if it is used in the reaction step.

Therefore, from an economic standpoint, it is preferable to subject the reaction mixture obtained from the carbonic acid monoester salt production step directly to the reaction step.

### 3. Other Steps

The production method according to this embodiment may include optional steps in addition to the reaction step. Examples of optional steps include a purification step for increasing the purity of the carbonic acid ester.

As a purification method for the carbonic acid diester in the purification step, purification methods commonly performed in the field of organic synthesis, such as filtration, adsorption, column chromatography, and distillation, can be employed. Specifically, examples include a method of filtering the obtained solid under a nitrogen atmosphere, washing it with diethyl ether or the like, and vacuum drying it.

### EXAMPLES

Hereinafter, the present disclosure will be described more specifically with reference to examples. However, the present disclosure can be appropriately modified without departing from the spirit thereof. Therefore, the scope of the present disclosure should not be construed as being limited by the specific examples shown below.

### <GC Measurement>

The measurement of product yield by gas chromatography (GC) was performed under the following conditions.
Apparatus name: GC-2014 (manufactured by Shimadzu Corporation)
Detector: FID (Flame Ionization Detector)
Column: TC-1 (manufactured by GL Sciences Inc.)
Carrier gas: N₂
Internal standard substance: Mesitylene
Data processing: Lab solutions (manufactured by Shimadzu Corporation)

### [Examples 1-1 to 1-3]

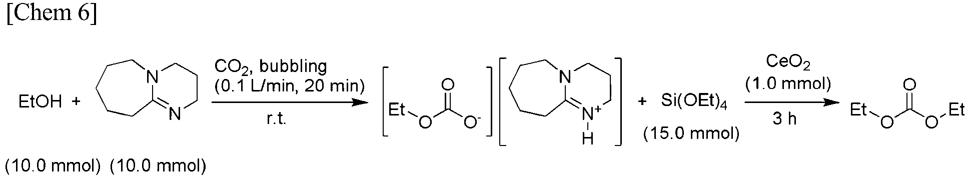

To a mixture of ethanol (0.58 mL, 10.0 mmol) and 1,8-diazabicyclo[5.4.0]undec-7-ene (1.52 g, 10.0 mmol), carbon dioxide gas was bubbled at a flow rate of 0.1 L/min for 20 minutes at room temperature, thereby preparing a mixed solution containing a carbonic acid monoester salt. This mixed solution, tetraethoxysilane (3.35 mL, 15.0 mmol), and cerium(IV) oxide (172 mg, 1.0 mmol) were placed in a sealed reactor and reacted for 3 hours at the reaction temperatures shown in Table 1, thereby obtaining diethyl carbonate. The yield of the obtained diethyl carbonate was calculated by GC measurement using mesitylene as an internal standard. The results are shown in Table 1.

### [Table 1]

**Table 1**

| | Reaction temperature (°C) | Yield of carbonic acid diester* (mmol) |
|---|---|---|
| Example 1-1 | 100 | 0.15 |
| Example 1-2 | 125 | 1.50 |
| Example 1-3 | 150 | 1.70 |

| | | |
|---|---|---|
| *: yield calculated by GC measurement using mesitylene as an internal standard | | |

From Table 1, it is evident that the reaction between the carbonic acid monoester salt and the alkoxysilane proceeds even at a low temperature of 100°C, yielding diethyl carbonate. Furthermore, Table 1 also shows that when the reaction temperature is increased from 100°C to 125°C, the yield of diethyl carbonate increases tenfold, and when further increased to 150°C, the yield of diethyl carbonate further improves.

### [Examples 2-1 to 2-4 and Comparative Examples 2-1 to 2-7]

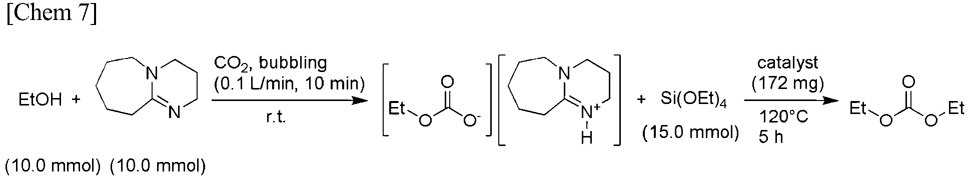

To a mixture of ethanol (0.58 mL, 10.0 mmol), 1,8-diazabicyclo[5.4.0]undec-7-ene (1.52 g, 10.0 mmol), tetraethoxysilane (3.35 mL, 15.0 mmol), and the catalyst (172 mg) shown in Table 2, carbon dioxide gas was bubbled at a flow rate of 0.1 L/min for 10 minutes at room temperature. Subsequently, diethyl carbonate was obtained by heating the resulting mixture at 120°C for 5 hours. The yield of the obtained diethyl carbonate was calculated by GC measurement using mesitylene as an internal standard. The results are shown in Table 2.

The catalyst used in the reaction was previously calcined in an air atmosphere at 600°C for 3 hours.

### [Table 2]

**Table 2**

| | Catalyst | Yield of carbonic acid diester^{*1} (mmol) |
|---|---|---|
| Example 2-1 | CeO₂ | 1.60 |
| Example 2-2 | CeO₂-ZrO₂^{*2} | slightly less than 0.075 |
| Example 2-3 | dibutyltin dilaurate | slightly less than 0.075 |
| Example 2-4 | dibutyltin oxide | slightly less than 0.075 |
| Comparative Example 2-1 | ZrO₂ | N.D.^{*5} |
| Comparative Example 2-2 | Al₂O₃^{*3} | N.D.^{*5} |
| Comparative Example 2-3 | TiO₂^{*4} | N.D.^{*5} |
| Comparative Example 2-4 | MgO | N.D.*⁵ |
| Comparative Example 2-5 | La₂O₃ | N.D.^{*5} |
| Comparative Example 2-6 | Ta₂O₅ | N.D.^{*5} |
| Comparative Example 2-7 | - | N.D.^{*5} |

| | | |
|---|---|---|
| *1: yield calculated by GC measurement using mesitylene as an internal standard *2: a mixture of CeO₂ and ZrO₂ containing 33 wt% CeO₂ *3: JRC-ALO-8 *4: JRC-TIO-13 *5: not detected | | |

From Table 2, it is evident that the reaction between the carbonic acid monoester salt and the alkoxysilane proceeds in the presence of catalysts such as cerium(IV) oxide, dibutyltin dilaurate, or dibutyltin oxide. That is, the above examples confirmed that cerium catalysts and tin catalysts exhibit catalytic activity in the reaction of a carbonic acid monoester salt with an alkoxysilane to produce a carbonic acid diester.

Furthermore, Table 2 also shows that cerium oxide exhibits particularly high catalytic activity in the reaction between the carbonic acid monoester salt and the alkoxysilane.

### [Examples 3-1 to 3-2]

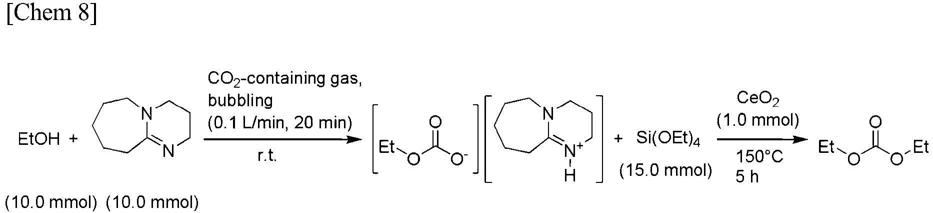

To a mixture of ethanol (0.58 mL, 10.0 mmol) and 1,8-diazabicyclo[5.4.0]undec-7-ene (1.52 g, 10.0 mmol), a mixed solution containing a carbonic acid monoester salt was prepared by bubbling a carbon dioxide-containing gas as shown in Table 3 at a flow rate of 0.1 L/min for 20 minutes at room temperature. This mixed solution, tetraethoxysilane (3.35 mL, 15.0 mmol), and cerium(IV) oxide (172 mg, 1.0 mmol) were placed in a sealed reactor and reacted at 150°C for 5 hours to obtain diethyl carbonate. The yield of the obtained diethyl carbonate was calculated by GC measurement using mesitylene as an internal standard. The results are shown in Table 3.

### [Example 3-3]

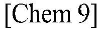

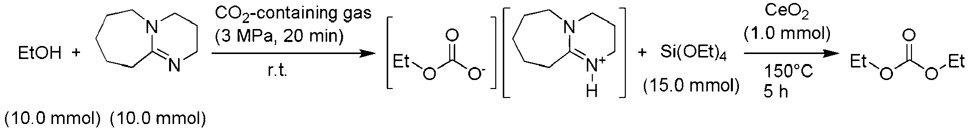

Ethanol (0.58 mL, 10.0 mmol) and 1,8-diazabicyclo[5.4.0]undec-7-ene (1.52 g, 10.0 mmol) were placed in a sealed reactor, and carbon dioxide gas was pressurized into this reactor such that the carbon dioxide pressure became 3.0 MPa. The mixture was allowed to react at room temperature for 20 minutes to prepare a mixed solution containing a carbonic acid monoester salt. Tetraethoxysilane (3.35 mL, 15.0 mmol) and cerium(IV) oxide (172 mg, 1.0 mmol) were added to this mixed solution, and the mixture was reacted at 150°C for 5 hours to obtain diethyl carbonate. The yield of the obtained diethyl carbonate was calculated by GC measurement using mesitylene as an internal standard. The results are shown in Table 3.

### [Table 3]

**Table 3**

| | CO₂-containing gas | Yield of carbonic acid diester^{*1} (mmol) |
|---|---|---|
| Example 3-1 | CO₂/N₂ (v/v=15/85)^{*2} | 1.7 |
| Example 3-2 | CO₂^{*3} | 1.6 |
| Example 3-3 | CO₂^{*4} | 2.0 |

| | | |
|---|---|---|
| *1: yield calculated by GC measurement using mesitylene as an internal standard *2: Pt=0.10MPa, P_{CO2}=0.015MPa, P_{CO2}/Pₜ=0.15 *3: Pₜ=0.10MPa, P_{CO2}=0.10MPa, P_{CO2}/Pₜ=1.00 *4: Pₜ=3.0MPa, P_{CO2}=3.0MPa, P_{CO2}/Pₜ=1.00 | | |

From Table 3, it can be inferred that in the reaction between an alcohol, a base, and a carbon dioxide-containing gas, when the carbon dioxide-containing gas was bubbled (P_total = 0.1 MPa), the yield of carbonic acid diester was hardly affected by the partial pressure of carbon dioxide gas in the carbon dioxide-containing gas. Therefore, if a sufficient amount of carbonic acid monoester salt can be formed by bubbling the carbon dioxide-containing gas for a sufficient period, carbonic acid diester can be obtained in high yield.

Furthermore, Table 3 also shows that in the reaction between an alcohol, a base, and a carbon dioxide-containing gas, when the partial pressure of carbon dioxide gas in the carbon dioxide-containing gas was 1.0, increasing the total pressure of the carbon dioxide-containing gas from 0.1 MPa to 3.0 MPa resulted in an increased yield of carbonic acid diester. This is expected because, in addition to an increased formation amount of carbonic acid monoester salt under high-pressure conditions, the dissolution of carbon dioxide into the reaction solution increases the amount of carbon dioxide in the reactor, shifting the reaction equilibrium towards the product side, thereby increasing the yield of carbonic acid diester.

### [Examples 4-1 to 4-3]

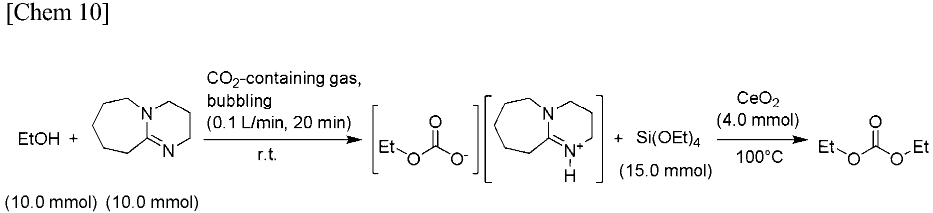

To a mixture of ethanol (0.58 mL, 10.0 mmol) and 1,8-diazabicyclo[5.4.0]undec-7-ene (1.52 g, 10.0 mmol), a mixed solution containing a carbonic acid monoester salt was prepared by bubbling a carbon dioxide-containing gas at a flow rate of 0.1 L/min for 20 minutes at room temperature. This mixed solution, tetraethoxysilane (3.35 mL, 15.0 mmol), and cerium(IV) oxide (682 mg, 4.0 mmol) were placed in a sealed reactor and reacted at 100°C for the times shown in Table 4 to obtain diethyl carbonate. The yield of the obtained diethyl carbonate was calculated by GC measurement using mesitylene as an internal standard. The results are shown in Table 4.

### [Table 4]

**Table 4**

| | CO₂-containing gas | Reaction time (h) | Yield of carbonic acid diester^{*1} (mmol) |
|---|---|---|---|
| Example 4-1 | CO₂/N₂ (v/v=15/85)*² | 26 | 1.7 |
| Example 4-2 | CO₂*³ | 26 | 2.6 |
| Example 4-3 | CO₂*³ | 48 | 3.5 |

| | | | |
|---|---|---|---|
| *1: yield calculated by GC measurement using mesitylene as an internal standard *2: Pₜ=0.1MPa, P_{CO2}=0.015MPa, P_{CO2}/Pₜ=0.15 *3: Pₜ=0.1MPa, P_{CO2}=0.10MPa, P_{CO2}/Pₜ=1.00 | | | |

From Table 4, it is inferred that in the reaction among an alcohol, a base, and a carbon dioxide-containing gas, when the carbon dioxide-containing gas was bubbled (P_total = 0.1 MPa), a higher partial pressure of carbon dioxide gas in the carbon dioxide-containing gas and a longer reaction time lead to the formation of carbonic acid monoester salt in higher yield, and as a result, diethyl carbonate is also obtained in high yield.

Furthermore, comparing Table 1 and Table 4, it can be seen that in the reaction between the carbonic acid monoester salt and the alkoxysilane, even under the low-temperature condition of 100°C, the yield of carbonic acid ester can be improved by increasing the amount of catalyst or extending the reaction time.

### [Examples 5-1 to 5-8]

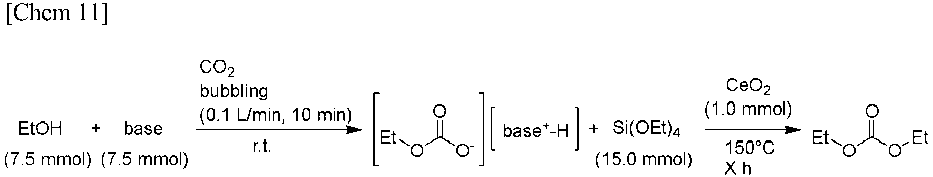

To a mixture of ethanol (0.44 mL, 7.5 mmol) and a base (7.5 mmol), a mixed solution containing a carbonic acid monoester salt was prepared by bubbling carbon dioxide gas at a flow rate of 0.1 L/min for 10 minutes at room temperature. This mixed solution, tetraethoxysilane (3.35 mL, 15.0 mmol), and cerium(IV) oxide (172 mg, 1.0 mmol) were placed in a sealed reactor and reacted at 150°C to obtain diethyl carbonate. The yield of the obtained diethyl carbonate was calculated by GC measurement using mesitylene as an internal standard. The results are shown in Table 5.

### [Table 5]

**Table 5**

| | Base | X (h) | Yield of carbonic acid diester* (mmol) |
|---|---|---|---|
| Example 5-1 | 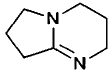 1,5-diazabicyclo[4.3.0]non-5-ene | 7 | 0.40 |
| Example 5-2 | | 48 | 0.40 |
| Example 5-3 | 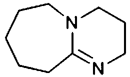 1,8-diazabicyclo[5.4.0]undec-7-ene | 7 | 1.05 |
| Example 5-4 | | 48 | 1.95 |
| Example 5-5 | 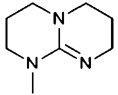 7-methyl-1,5,7-triazabicyclo[4.4.0]dec-5-ene | 7 | 0.60 |
| Example 5-6 | | 48 | 1.50 |
| Example 5-7 | 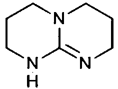 1,5,7-triazabicyclo[4.4.0]dec-5-ene | 7 | 0.35 |
| Example 5-8 | | 48 | 0.40 |

| | | | |
|---|---|---|---|
| *: yield calculated by GC measurement using mesitylene as an internal standard | | | |

From Table 5, it is evident that salts of a carbonic acid monoester with various cationic species can be utilized as the carbonic acid monoester salt for producing carbonic acid diester by reacting a carbonic acid monoester salt with an alkoxysilane. Furthermore, among the carbonic acid monoester salts, it is understood that carbonic acid diester can be efficiently obtained, particularly by using carbonic acid monoester amidinium salts and carbonic acid monoester guanidinium salts as the carbonic acid monoester salt.

### [Example 6-1]

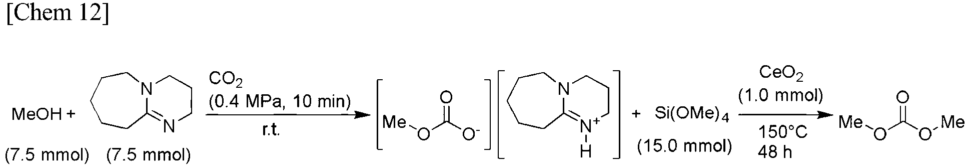

Methanol (0.30 mL, 7.5 mmol) and 1,8-diazabicyclo[5.4.0]undec-7-ene (1.14 g, 7.5 mmol) were placed in a sealed reactor, and carbon dioxide gas was pressurized into this reactor such that the carbon dioxide pressure became 0.4 MPa. The mixture was allowed to react for 20 minutes at room temperature, thereby preparing a mixed solution containing a carbonic acid monoester salt. Tetramethoxysilane (2.22 mL, 15.0 mmol) and cerium(IV) oxide (172 mg, 1.0 mmol) were added to this mixed solution, and dimethyl carbonate was obtained by reacting at 150°C for 48 hours. The yield of the obtained dimethyl carbonate was calculated by GC measurement using mesitylene as an internal standard. The results are shown in Table 6.

### [Example 6-2]

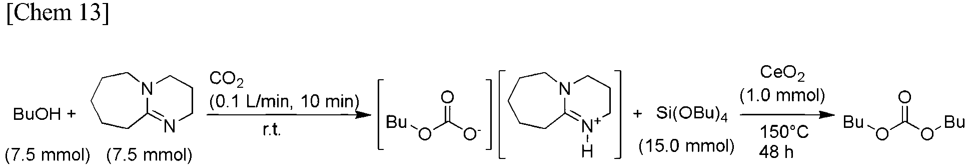

To a mixture of 1-butanol (0.66 mL, 7.5 mmol) and 1,8-diazabicyclo[5.4.0]undec-7-ene (1.14 g, 7.5 mmol), a mixed solution containing a carbonic acid monoester salt was prepared by bubbling carbon dioxide gas at a flow rate of 0.1 L/min for 10 minutes at room temperature. This mixed solution, tetrabutoxysilane (5.35 mL, 15.0 mmol), and cerium(IV) oxide (172 mg, 1.0 mmol) were placed in a sealed reactor, and dibutyl carbonate was obtained by reacting at 150°C for 48 hours. The yield of the obtained dibutyl carbonate was calculated by GC measurement using mesitylene as an internal standard. The results are shown in Table 6.

### [Table 6]

**Table 6**

| | Alcohol used in carbonic acid monoester salt production step | Alkoxysilane used in reaction step | Yield of carbonic acid diester* (mmol) |
|---|---|---|---|
| Example 6-1 | methanol | tetramethoxysilane | 1.15 |
| Example 6-2 | 1-butanol | tetrabutoxysilane | 0.10 |

| | | | |
|---|---|---|---|
| *: yield calculated by GC measurement using mesitylene as an internal standard | | | |

### INDUSTRIAL APPLICABILITY

According to the present invention, a carbonic acid diester can be produced by reacting a carbonic acid monoester salt with an alkoxysilane in the presence of one or more catalysts selected from the group consisting of a cerium catalyst and a tin catalyst, without using sacrificial reagents such as alkyl halides and without phosgene.

## Claims

1. A method for producing a carbonic acid diester, comprising a reaction step of reacting a carbonic acid monoester salt with an alkoxysilane in the presence of one or more catalysts selected from the group consisting of a cerium catalyst and a tin catalyst.

2. The method for producing the carbonic acid diester according to claim 1, wherein the carbonic acid monoester salt is a compound represented by general formula (1), the alkoxysilane is a compound represented by general formula (2), and the carbonic acid diester is a compound represented by general formula (3): 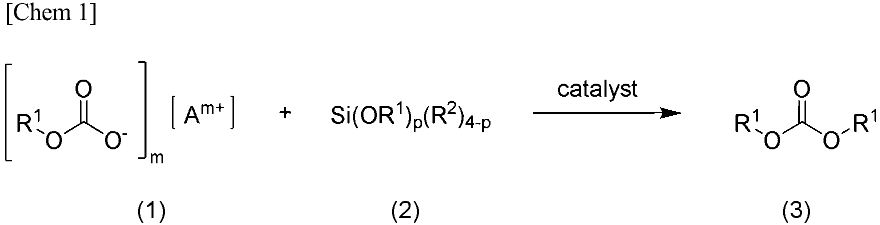 wherein in the general formulae, R¹ each independently is a substituted or unsubstituted hydrocarbon group; R² each independently is a substituted or unsubstituted hydrocarbon group; A^{m+} is an m-valent cation derived from a base represented by A; m is 1 or 2; p is an integer of 1 to 4; and when p is 1 or 2, the plurality of R² may be linked to each other to form a ring.

3. The method for producing the carbonic acid diester according to claim 2, wherein R¹ is a substituted or unsubstituted aliphatic hydrocarbon group.

4. The method for producing the carbonic acid diester according to claim 2 or 3, wherein p is 2 to 4.

5. The method for producing the carbonic acid diester according to any one of claims 1 to 4, wherein the reaction step is performed under a temperature condition of 80°C or more and 250°C or less.

6. The method for producing the carbonic acid diester according to any one of claims 1 to 5, wherein the catalyst is one or more selected from the group consisting of cerium (IV) oxide, a dialkyltin bis(aliphatic monocarbonate), and a dialkyltin oxide.

7. The method for producing the carbonic acid diester according to any one of claims 1 to 6, comprising, prior to the reaction step, a carbonic acid monoester salt production step of contacting an alcohol, a base, and a carbon dioxide-containing gas,
wherein a ratio of partial pressure P_{CO2} of carbon dioxide gas in the carbon dioxide-containing gas to total pressure Pₜ of the carbon dioxide-containing gas is 0.0001 or more and 1.00 or less.

8. The method for producing the carbonic acid diester according to claim 7, wherein the partial pressure P_{CO2} of carbon dioxide gas in the carbon dioxide-containing gas is less than 0.100 MPa.
